Europäisches Patentamt

European Patent Office

Office européen des brevets

(11) Publication number: **0 280 054 B1**

# EUROPEAN PATENT SPECIFICATION

(45) Date of publication of patent specification: **11.11.92** (51) Int. Cl.⁵: **C12N 5/02**

(21) Application number: **88101138.1**

(22) Date of filing: **27.01.88**

(54) **Improved process for preparing activated killer cells.**

(30) Priority: **29.01.87 US 8273**

(43) Date of publication of application:
**31.08.88 Bulletin 88/35**

(45) Publication of the grant of the patent:
**11.11.92 Bulletin 92/46**

(84) Designated Contracting States:
**AT BE CH DE ES FR GB GR IT LI LU NL SE**

(56) References cited:
**GB-A- 2 065 067**
**US-A- 4 140 162**
**US-A- 4 280 497**
**US-A- 4 496 361**
**US-A- 4 588 401**

**CANCER, vol. 55, 15th March 1985, pages 1327-1333; A.A. RAYNER et al.: "Lymphokine-activated killer (LAK) cells. Analysis of factors relevant to the immunotherapy of human cancer"**

**JOURNAL OF IMMUNOLOGICAL METHODS, vol. 88, 1986, pages 265-275; L. MESLER MUUL et al.: "Large scale production of human lymphokine activated killer cells for use in adoptive immunotherapy"**

(73) Proprietor: **TERUMO Kabushiki Kaisha**
**44-1 Hatagaya 2-chome Shibuya-ku**
**Tokyo(JP)**

(72) Inventor: **Irr, Joseph David**
**386 Briar Lane**
**Newark Delaware 19711(US)**

(74) Representative: **von Kreisler, Alek et al**
**Patentanwälte Von Kreisler-**
**Schönwald-Fues-Keller Selting-Werner**
**Deichmannhaus am Hauptbahnhof**
**W-5000 Köln 1(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

**BACKGROUND OF THE INVENTION**

Field of the Invention

This invention relates to a process for preparing activated killer cells, particularly lymphokine activated killer (LAK) cells.

Description of Related Art

Rayner, et al., Cancer 55, 1327-1333 (1985), disclose that lymphokine-activated killer (LAK) cells can be generated by incubating fresh peripheral blood lymphocytes in interleukin-2. The authors disclose that LAK cells kill fresh autologous and allogeneic human tumor cells in vitro. The LAK cells can be generated from peripheral blood lymphocytes of normal individuals and tumor-bearing patients. They report that pure, recombinant interleukin-2 (rIL-2) generates LAK cells capable of killing a wide variety of tumors including sarcomas and cancers of the colon, pancrease, adrenal gland, and esophagus. They report that the use of LAK cells, possibly with the systemic administration of rIL-2, represents a promising future approach to the immunotherapy of human cancer. The reference discloses activation of the peripheral blood lymphocytes in 25-cm$^2$ flasks at a cell concentration of $10^6$ cells/mL for 5 days in 5% $CO_2$ at 37° C.

Rosenberg, et al., The New England Journal of Medicine 313, 1485-1492 (1985), disclose preliminary results of the systemic administration of autologous lymphokine-activated killer cells and rIL-2 to patients with advanced cancer. They treated 25 patients with metastatic cancer in whom standard therapy had failed. Objective regression of cancer (more than 50 percent of volume) was observed in 11 of the 25 patients. Complete tumor regression occurred in one patient with metastatic melanoma and had been sustained for up to 10 months after therapy. The reference discloses activation or culturing of the cells in 2.5-liter roller bottles at a cell concentration of $1.5 \times 10^6$ cells/mL for 3-4 days at 37° C. Patients were infused with up to about $2 \times 10^{11}$ LAK cells.

Rosenberg, et al., Science 233, 1318-1321 (1986), disclose that adoptive transfer of tumor-infiltrating lymphocytes (TIL) expanded in interleukin-2 to mice bearing micrometastases from various types of tumors showed that TIL are 50 to 100 times more effective in their therapeutic potency than LAK cells. The TIL were prepared by harvesting tumors aseptically and mincing them into 1-2 mm pieces which were then stirred in 40 mL of a specified saline solution to obtain a cell suspension which was filtered, washed and suspended in complete medium containing rIL-2 until about a 100-fold expansion of the original cells was obtained.

U.S. Patent 3,928,294, issued on December 23, 1975 to Crawford, et al., discloses a permselective, biocompatible membrane article having high gaseous permeability, said membrane consisting of a poly-(alpha-olefin-sulfone) derived from the series $C_8$-$C_{18}$ alpha-olefins and sulfur dioxide, the membrane having an oxygen transmission rate of about 1 to $4 \times 10^4$ cc mil/in$^2$ yr atm and a $CO_2$ transmission rate of about 4 to $17 \times 10^4$ cc mil/in$^2$ yr atm. Articles which can be fabricated from the membrane material include blood storage bags.

Belgian Patent 862,772 discloses a medical device wherein at least the part which comes into contact with a bodily fluid and/or a liquid medication is made from a copolymer which is crosslinked 20 to 75% by irradiation and has a specified formula. The copolymer is prepared by polymerization of an ester having an acrylic radical and an ethylene compound, both of a specified formula. Included among the devices disclosed is a blood bottle. The patent is directed to materials having low permeability to gases.

U.S. Patents 4,496,361 and 4,588,401, issued to Kilkson on January 29, 1985 and May 13, 1986, respectively, disclose a platelet storage container made of a copolymeric film material capable of being heat sealed at a low temperature and having

an oxygen permeability of from about $1.8 \times 10^5$ $\mu$m$^3$ (STP)/(m$^2 \cdot$ sec $\cdot$ Pa) [100 cc (STP)/(24 hr-atm-100 in$^2$)],

a carbon dioxide permeability of from about $4.4 \times 10^5$ to about $8.0 \times 10^5$ $\mu$m$^3$ (STP)/(m$^2 \cdot$ sec $\cdot$ Pa) [250 to about 450 cc (STP)/(24 hr-atm-100 in$^2$)],

a tensile strength of at least about 8 mPa,

a seal strength of at least about 1000 g/cm,

a stiffness low enough so that the container can be extended by liquid to a capacity of about 275 mL,

a durability sufficient to provide a dart drop value of at least about 100, and

a thickness of from about 0.08 mm to about 0.23 mm.

EP 0 280 054 B1

Blood is stored at 4° C and platelets are stored at about 22° C.

U.K. Patent Application Publication 2,065,067 discloses a bag for use in freezing physiological solution, food or chemicals comprising a laminated sheet heat sealed to form a bag, the sheet comprising an inner layer of unstretched film of a random copolymer of an α-olefin with ethylene containing from 1 to 20 mol % of α-olefin of over $1 \times 10^4$ average molecular weight, having a density less than 0.936 and having 4 to 18 carbon atoms, and an outer layer of a heat-resistant polymerized film whose glass transition temperature is above room temperature. The α-olefin can be, amongst others, 1-butene, 4-methyl-1-pentene, or 1-octene, and the outer layer can be materials such as ethylene-tetrafluorethylene copolymer.

In the effort to further investigate and refine LAK cells, TIL and rIL-2 as an adoptive immunotherapies for cancer, improvements which allow activation at a higher concentration of cells or otherwise increased attractiveness of the therapies are most desirable. For example, culturing at a concentration of about $1.5 \times 10^6$ cells/mL when about $1.8 \times 10^{10}$ to $1.8 \times 10^{11}$ cells are needed would require total culture volumes of from about 12 to 120 L. The benefits of a technique which would permit the use of signficantly lower volumes are apparent.

## SUMMARY OF THE INVENTION

The present invention provides an improved process for preparing lymphokine activated killer (LAK) cells or TIL. In a process wherein peripheral blood mononuclear cells or lymphocytes from tumor-tissue are cultured to produce a population of cells which are cytotoxic for natural killer cell resistant tumor cells, the improvement comprises culturing a population of cells selected from the group consisting of peripheral blood mononuclear cells, peripheral blood lymphocytes resulting therefrom, and lymphocytes obtained from tumor tissue in a closed container made from a copolymeric film material having an oxygen permeability of at least about $1.8 \times 10^5$ $\mu m^3$ (STP)/(M²•sec•Pa); and a thickness of from about 0.04 mm to about 0.23 mm; said material being selected from the group consisting of

(a) copolymers of ethylene and an α-olefin of 4-10 carbon atoms having a density of from about 0.915-0.925 g/cm³;

(b) copolymers of ethylene and methacrylic acid;

(c) ionomers; and

(d) laminates or coextrudates of an ionomer/polyester elastomer and a linear low density polyethylene elastomer.

## DETAILED DESCRIPTION OF THE INVENTION

As used herein:

"Interleukin-2" (IL-2) means human interleukin-2 which is a glycoprotein with a molecular weight of approximately 15,000 daltons and consists of a 133 amino acid polypeptide containing a disulfide bridge. The term, as used herein, includes natural and recombinant interleukin-2 (rIL-2) and biologically functional equivalents thereof. "Biologically functional equivalents" mean polypeptides having the same or very similar biological activity, such as rIL-2 muteins described in U.S. Patent 4,518,584 and rIL-2 proteins having a methionine replacing the $NH_2$-terminal alanine found in native IL-2. The disclosure of U.S. Patent 4,518,584 in regard to rIL-2 muteins is incorporated herein by reference. These muteins are rIL-2 molecules wherein the cysteine at position 125, numbered in accordance with native human IL-2, is deleted or replaced by a neutral amino acid and said mutein exhibits the bilogical activity of native human IL-2.

"Lymphokine activated killer (LAK) cells" means a cytotoxid population of cells which are capable of lysing autologous tumor cells and natural killer (NK) cell resistant tumor cell lines and are generally prepared by culturing peripheral blood mononuclear cells with interleukin-2.

"Peripheral blood lymphocytes" means peripheral blood mononuclear cells from which monocytes have been depleted.

"Lower alkyl" means alkyl of 1-4 carbon atoms.

"Copolymeric film material" means a film material in which each layer is made from a single copolymer or homopolymer and at least one layer is made from a single copolymer.

"STP" means standard temperature and pressure.

"RPMI 1640" refers to a culture medium which is well known in the art and whose composition is given in "Culture of Animal Cells", Freshney, 72-73, Alan R. Liss, Inc., N.Y.

The process of the invention enables LAK-cell activation at concentrations of up to about $5 \times 10^7$ cells/mL without altering the extent or range of LAK-cell activity. At these cells for adoptive immunotherapy can be obtained with culture volumes of from about 0.2 to about 2 L. Although the invention will be described in

3

specific with reference to human peripheral blood mononuclear cells or human peripheral blood lymphocytes, it is to be understood that the invention applies to the corresponding cells of other mammals. Preferably, the process of the invention is used with human cells. The process of the invention is also useful for activation of TIL.

Peripheral blood mononuclear cells (PBMC) required for the process of the invention are obtained by repeated leukapheresis using procedures well known in the art to collect up to about $5 \times 10^{10}$ mononuclear cells. In general, blood is removed intravenously from a human donor and passed to an instrument which separates mononuclear cells from red blood cells, platelets and plasma, these latter components being returned to the donor. The mononuclear cells are collected in a plastic bag. The PBMC so obtained are fractionated by Ficoll-Hypaque density gradient separation. The cells are then washed with a suitable salt solution, such as Hanks' balanced salt solution (HBSS), which is obtainable from Gibco, Grand Island, NY, and then resuspended in a suitable medium, such as RPMI 1640 medium, which is also obtainable from Gibco, supplemented with 1 mM L-glutamine solution, 1% penicillin-streptomycin solution, 1% gentamycin solution, and 2-10% heat inactivated pooled human serum (HIS) (percentages are v/v unless otherwise specified).

The resuspended PBMC are preferably, prior to density gradient separation, treated with an L-amino acid lower alkyl ester or hydrogen chloride salt thereof. This optional treatment can also be effected after density gradient separation. Suitable L-amino acid lower alkyl esters are those wherein the amino acid is selected from phenylalanine, glutamic acid, glutamine and tyrosin. Preferably, the L-amino acid is phenylalanine or tyrosine and most preferably is phenylalanine. The lower alkyl group can be methyl, ethyl, propyl, isopropyl, butyl, isobutyl or t-butyl but is preferably methyl or ethyl and is most preferably methyl. The PBMC can be resuspended in any suitable medium such as serumless RPMI 1640 or HBSS without Ca and Mg, but the latter is preferred. Preferably, the hydrogen chloride salt of the L-amino acid lower alkyl ester is dissolved in RPMI and the pH of the resulting solution is adjusted to about 7.4 prior to adding the resulting solution to the suspension of PBMC. The amino acid lower alkyl ester is present at a concentration of from about 1 mM to about 5 mM (based on the total volume of the combined PBMC suspension and the amino acid lower alkyl ester solution). Contacting of the PBMC and ester is preferably carried out at a temperature of from 20° C to about 25° C. Treatment of the PBMC with L-amino acid lower alkyl ester causes depletion of monocytes and also confers on the ultimate LAK cell product enhanced activity after culturing with IL-2 at higher cell concentration. This L-amino acid lower alkyl ester treatment is further described in assignee's copending U.S. patent application Serial No. 868,697, filed on May 30, 1986, which is incorporated herein by reference. Monocytes can also be depleted from PBMC by using conventional techniques such as the passage of the PMBC over glass beads. The resulting peripheral blood lymphocytes obtained in this manner are preferably then treated with the amino acid lower alkyl ester as set forth above.

In the process of the invention the peripheral blood mononuclear cells or peripheral blood lymphocytes resulting therefrom are cultured with IL-2 for an incubation period of from about 2 to 7 days, perferably about 3 to 5 days, preferably at a temperature of from about 35° C to about 39° C, most preferably 37° C. Culturing is conducted in a closed container made from a copolymeric film material having an oxygen permeability of at least about $1.8 \times 10^5$ $\mu m^3$ (STP)/($m^2 \cdot sec \cdot Pa$) [100 cc (STP)/(24 hr-atm-100 in$^2$)]; and a thickness of from about 0.04 mm to about 0.23 mm. The copolymeric material is selected from the group consisting of

(a) copolymers of ethylene and an $\alpha$-olefin of 4-10 carbon atoms having a density of from abot 0.915-0.925 g/cm$^3$ (a so-called linear low density polyethylene); typical $\alpha$-olefins include 1-butene, hexene, 1-octene and 1-decene;

(b) copolymers of ethylene and methacrylic acid;

(c) ionomers, such as sodium or zinc neutralized copolymers of ethylene and acrylic or methacrylic acid or derivatives thereof; and

(d) laminates or coextrudates of an ionomer/polyester elastomer and a linear low density polyethylene elastomer. Preferably, the copolymeric film material is a copolymer of ethylene and an $\alpha$-olefin of 4-10 carbon atoms having a density of from about 0.915-0.925 g/cm$^3$. These copolymers of ethylene and an $\alpha$-olefin of 4-10 carbon atoms have about 3-5 mol percent of the latter component. Preferably the $\alpha$-olefin is 1-butene or 1-octene, most preferably 1-octene. Suitable copolymeric materials should be capable of being heat sealed at a low temperature, i.e., from about 125° C to about 140° C. Fabrication of containers is facilitated by materials which meet this requirement.

The thickness of the copolymeric film material selected for use in constructing the container selected for use in constructing the container will depend on the permeability per millimeter and the mechanical durability of the copolymeric material. For instance, if the material has relatively high oxygen permeability, then a relatively high thickness can be used. On the other hand, if the oxygen permeability of the material at a particular thickness is marginally low while the mechanical durability is rather high, than a lower thickness can be employed. For a copolymer of ethylene and 1-butene or 1-octene having a density of from 0.915 to 0.925 g/cm$^3$, the film material will have a thickness of from abut 0.04 mm to about 0.14 mm. The copolymeric film material should be sufficiently mechanically durable to withstand the moderate stresses encounter during use and is preferably translucent. The container should have sufficient volume to accommodate from about 0.2 to about 5 liters of cell suspension. Preferably, the container is a flexible bag. The container is preferably equipped with suitable access ports or tubes which can be closed by methods well known in the art, such as by use of clamps, etc., to maintain a reasonable level of protection from contamination. In a preferred embodiment the tubes are closed by heat sealing.

The copolymeric film material must also be compatible with the PBMC or PBL and the culture medium. By "compatible" it is meant that the material does not interact with the cells or medium or leach into them chemicals which are deleterious to the cells or recipient.

In the process of the invention the PBMC or peripheral blood lymphocytes (PBL) in suspension are placed in the container along with suitable medium and IL-2, the container is closed to maintain sterility, and the resulting suspension is incubated for the time period prescribed earlier herein. The container holding the PBMC or PBL, culture medium and IL-2 is preferably maintained in the presence of a from about 3% to about 10% by volume CO$_2$ atmosphere, most perferably 5% CO$_2$. The PBMC or PBL are preferably suspended at a cell concentration during culturing of from about $1.0 \times 10^6$ to about $5 \times 10^7$ cells/mL, most preferably from about $5 \times 10^6$ to about $1.5 \times 10^7$ cells/mL. Suitable media include RPMI 1640, supplemented with serum or with human serum albumin (HSA). Preferably, the medium is RPMI supplemented with HSA, most preferably supplemented with the therapeutic grade HSA. Preferably, the IL-2 concentration is from about $5 \times 10^2$ to about $5 \times 10^4$ pM , most preferably, from 1000 pM to about 2000 pM. Preferably, the IL-2 is rIL-2, and, most preferably, the rIL-2 is a rIL-2 composition consisting essentially of water, rIL-2, and optionally, a polyol, said composition having an IL-2 specific activity of at least about 120,000 units/mg, said specific activity being at least 40% of that of Jurkat IL-2. This rIL-2 composition is described in copending U.S. patent application Serial No. 825,133, filed on January 31, 1986 and assigned to E. I. du Pont de Nemours and Company. The relevant disclosure of this application is incorporated herein by reference. This rIL-2 composition is prepared by a process comprising (a) mixing with water to generate a suspension rIL-2 which has been lyophilized after purification by high performance liquid chromatography, and (b) heating the resulting suspension at a temperature of from about 25° C to about 95° C for at least about two hours.

In the process of the invention, during culturing the cell suspension is maintained at a depth that will allow adequate oxygen to reach all the cells. For instance, when the container is fabricated in the form of a blood bag, the bag is maintained in a horizontal position on a rack that will allow gases to enter the side contacting the rack. In this situation, if the cell density is low, the suspension depth can be up to about 4 cm; whereas, if the cell density is high the depth will be about 0.5 cm or less.

Previously hereto, the PBMC or PBL were cultured in conventional rigid containers, such as roller bottles, T-flasks, petri dishes, cluster dishes and polypropylene tubes. In a preferred embodiment of the invention the container used for culturing has one or more access tubes which are heat-sealed shut prior to sterilization of the container. In this embodiment, access to the container is obtained through use of a sterile connection device such as than described in U.S. Patent 4,369,779. The PBMC or PBL, culture medium and IL-2 are placed in the container through an access tube which is then sealed shut by use of the sterile connection device. After the incubation period, the LAK cells can be removed by using the sterile connection device to connect the access tube to the desired transfer container. In this manner sterility of the system can be maintained.

The LAK cells prepared by the process of the invention can be suspended in a pharmaceutically acceptable carrier, such as saline, saline containing 5% normal human serum albumin, or Hank's balanced salt solution, to provide a composition which can be infused into a patient afflicted with a tumor. The patient is concurrently treated with rIL-2 as further described by Rosenberg, et al., The New England Journal of Medicine 313, 1485-1492 (1985). In that modality, the patient's blood is withdrawn, subjected to leukapheresis and harvested cells are immediately cultured for 3 days to generate LAK cells. The LAK cells are then infused into the patient. Typically, about $3 \times 10^{10}$ to $14 \times 10^{10}$ LAK cells are infused in 4-9 doses. Interleukin-2 is administered every eight hours at doses such as 10,000, 30,000 or 100,000 units per kilogram of weight. The treatment consists of a two-week regime of leukapheresis and reinfusion and generally repetition starting the third week. Recombinant IL-2 can be included in the LAK cell composition.

Although the process of the invention has been described with specific reference to LAK cells, it is also useful for preparing TIL. These cells can be obtained by the procedure described in Rosenberg, et al., Science 233, 1318-1321 (1986). In this procedure a suspension of cells is obtained from 1-to 2-mm pieces of tumor tissue. This cell suspension can be then cultured persuant to the process of the invention but without the optional treatment with an L-amino acid lower alkyl ester.

The present invention can be carried out by the herein-described specific embodiments but is not limited thereto. The invention is further illustrated by the following examples in which all percentages are by volume and temperatures are in degrees Celsius, unless otherwise stated.

Cytotoxicity Assay

Unless otherwise stated, a 4 hour $^{51}$Cr release assay was used to measure cytotoxicity of LAK cells for tumor cells. Tumor cells at a concentration of about $2 \times 10^6$ to $10 \times 10^6$ were incubated with 100 $\mu$Ci of $Na_2^{51}CrO_4$ in 0.4 mL of Tris-phosphate buffered saline for 1 hour at 37°. The cells were washed 3 times with RPMI 1640 containing 5% or 10% fetal calf serum (FCS) and resuspended to $10^5$ cells/mL in RMPI-20% FCS or RPMI-10% FCS. The effector cells (LAK cells) were suspended to various concentrations and 0.1 mL was added to wells in round bottom microliter plates. The $^{51}$Cr labelled target cells (0.1 mL) were added to all wells and the plates were centrifuged at 200 xg for 5 minutes. After 4 hours of incubation at 37°, the plates were centrifuged again and 0.1 mL of resulting supernatant was removed from each well and counted in a gamma counter. Percent cytotoxicity is calculated from the following formula:

$$\text{\%cytotoxicity=} \frac{\text{experimental cpm} - \text{spontaneous cpm}}{\text{total cpm} - \text{spontaneous cmp}} \times 100$$

Each variable was tested in triplicate and the resulting data are expressed at % cytotoxicity. This cytotoxicity test is further described in "Selected Methods in Cellular Immunology", Mishell and Shiigi, eds., 124-137, W. H. Freeman and Co., San Franciso (1980).

In other experiments, the results of the assays are presented as "Lytic Units" (LU or LU30) which are the number of target cells per 100 effector cells when 30% of the target cells are killed when LAK cells and target cells are incubated together for 4 hours at 37° C. The calculation of LU is based upon the method of Pross, et al., Journal of Immunological Methods 68, 235-249 (1984). The greater the number of LU, the greater the potency of the LAK cell preparation.

## EXAMPLES 1-9 AND COMPARISONS A-F

PBMC were obtained by Ficoll-Hypaque separation of ACD-anticoagulated venus blood drawn from two healthy consenting human donors. Cells taken from one donor were treated with L-phenylalanine methyl ester whereas cells from the other donor were not. The methyl ester treatment was effected by washing the cells three times with HBSS and resuspending the resulting washed cells in RPMI 1640 medium supplemented with 1 mL L-glutamine, 0.1% gentamycin solution, 10% FCS amd 5 mM L-phenylalanine methyl ester. The resulting cell suspension was incubated at ambient temperature (about 22° C) for 40 minutes after which the cells were washed three times with HBSS.

The PBMC or PBL resulting from monocyte depletion with L-phenylalanine methyl ester were cultured in 10 mL of RPMI 1640 supplemented with 10% FCS in T Flask (T-25) (Comparisons) or closed bags constructed of a copolymer of ethylene (about 97 mol %) and 1-octene (LLDPE) having a density of 0.918 g/cm$^3$ and a nominal volume of 200 mL. A 0.12 mm thick LLDPE material of similar composition is known from U.S. Patent 4,496,361, Example 5, to have an oxygen permeability of $2.6 \times 10^5$ $\mu$m$^3$ (STP)/m$^2 \cdot$sec$\cdot$Pa). Culturing was conducted in 5% $CO_2$ atmosphere at 37° in the presence of 10 units/mL or rIL-2. One unit of IL-2 is defined as that quantity of IL-2 needed to stimulate 50% uptake of radiolabelled (tritium) thymidine by murine tumor-specific cytotoxic T-cell lines (CTLL) standardized against Jurkat IL-2 as set forth more fully in Gehmans, et al., Journal of Immunological Methods 74, 39-47 (1984), which is incorporated herein by reference.

After the culture period, the resulting LAK cells were harvested and resuspended in RPMI supplemented with 10% FCS for cytotoxicity assay using as target cells (T) Daudi cells (D) (an NK resistant cell line), melanoma cells (M), and lymphoma cells (L). The results are presented in Table 1 where the "E" refers to "effector cells", i.e., LAK cells. Experiments where the PBMC were treated with phenylalanine methyl ester (PME) are so indicated.

## TABLE 1

| Example No. or Comparison | PME | T | Cell Conc. (no./mL) $x10^{-6}$ | E:T Ratio | % $^{51}$Cr Release |
|---|---|---|---|---|---|
| 1 | no | D | 1.5 | 20:1 | 75.6 |
|   |    |   |     | 10:1 | 71.7 |
|   |    |   |     | 5:1  | 59.2 |
|   |    |   |     | 2.5:1 | 44.6 |
| 2 | no | D | 4 | 20:1 | 73.8 |

|  |  |  |  | 10:1 | 68.3 |
|---|---|---|---|---|---|
|  |  |  |  | 5:1 | 48.7 |
|  |  |  |  | 2.5:1 | 33.0 |
| 3 | yes | D | 5 | 20:1 | 82.2 |
|  |  |  |  | 10:1 | 73.9 |
|  |  |  |  | 5:1 | 57.2 |
|  |  |  |  | 2.5:1 | 35.5 |
| Comp. A | no | D | 1.5 | 20:1 | 80.5 |
|  |  |  |  | 10:1 | 73.9 |
|  |  |  |  | 5:1 | 60.1 |
|  |  |  |  | 2.5:1 | 44.5 |
| Comp. B | yes | D | 1.5 | 20:1 | 72.3 |
|  |  |  |  | 10:1 | 63.0 |
|  |  |  |  | 5:1 | 44.1 |
|  |  |  |  | 2.5:1 | 23.3 |
| 4 | no | M | 1.5 | 20:1 | 54.7 |
|  |  |  |  | 10:1 | 37.2 |
|  |  |  |  | 5:1 | 39.4 |
|  |  |  |  | 2.5:1 | 27.1 |
| 5 | no | M | 4 | 20:1 | 49.2 |
|  |  |  |  | 10:1 | 48.6 |
|  |  |  |  | 5:1 | 35.5 |
|  |  |  |  | 2.5:1 | 20.5 |
| 6 | yes | M | 5 | 20:1 | 34.0 |
|  |  |  |  | 10:1 | 25.6 |
|  |  |  |  | 5:1 | 25.5 |
|  |  |  |  | 2.5:1 | 17.7 |
| Comp. C | no | M | 1.5 | 20:1 | 44.4 |
|  |  |  |  | 10:1 | 31.1 |
|  |  |  |  | 5:1 | 25.6 |
|  |  |  |  | 2.5:1 | 17.7 |
| Comp. D | yes | M | 1.5 | 20:1 | 29.8 |
|  |  |  |  | 10:1 | 23.1 |
|  |  |  |  | 5:1 | 10.0 |
|  |  |  |  | 2.5:1 | 9.2 |
| 7 | no | L | 1.5 | 20:1 | 41.7 |

|  |  |  |  | 10:1 | 35.1 |
|---|---|---|---|---|---|
|  |  |  |  | 5:1 | 25.4 |
|  |  |  |  | 2.5:1 | 17.9 |
| 8 | no | L | 4 | 20:1 | 49.0 |
|  |  |  |  | 10:1 | 36.2 |
|  |  |  |  | 5:1 | 24.7 |
|  |  |  |  | 2.5:1 | 15.3 |
| 9 | yes | L | 5 | 20:1 | 33.1 |
|  |  |  |  | 10:1 | 32.2 |
|  |  |  |  | 5:1 | 19.2 |
|  |  |  |  | 2.5:1 | 7.8 |
| Comp. E | no | L | 1.5 | 20:1 | 46.1 |
|  |  |  |  | 10:1 | 39.4 |
|  |  |  |  | 5:1 | 31.3 |
|  |  |  |  | 2.5:1 | 23.2 |
| Comp. F | yes | L | 1.5 | 20:1 | 28.4 |
|  |  |  |  | 10:1 | 21.2 |
|  |  |  |  | 5:1 | 14.0 |
|  |  |  |  | 2.5:1 | 17.5 |

## EXAMPLE 10-12 AND COMPARISON G

PBMC were collected from healthy consenting human donors by means of either leukapheresis or American Red Cross whole blood specimens. In both cases the PBMC were obtained from venous blood prevented from clotting by means of ACD anticoagulant. The PBMC were separated from other blood components by Ficoll-Hypaque density gradient centrifugation. Some of PBMC preparations were treated with L-phenylalanine methyl ester (PME) to deplete them of monocytes. The methyl ester treatment was accomplished by washing the cells three times with HBSS and resuspending the resulting washed cells in RPMI 1640 medium supplemented with 1 mM L-glutamine , 0.1% gentamycin solution, 10% FCS and 5 mM L-phenylalanine methyl ester. The cell suspension so obtained was incubated at ambient temperature for 40 minutes after which the resulting cells were washed three times with HBSS and resuspended in RPMI 1640 containing L-glutamine, gentamycin and 10% FCS.

The PBMC or PBL resulting from monocyte depletion with methyl ester were cultured in 10 mL T-flask (Comparison G) or in closed bags constructed from a film of a copolymer of ethylene (97 mol %) and 1-octene (LLDPE) having a density of 0.918 $g/cm^3$, a nominal volume of 200 mL, and a film thickness of 0.13 mm (5 mil). Culturing was conducted in 5% $CO_2$ atmosphere at 37° C in the presence of 10 units/mL of rIL-2.

After the culture period the resulting LAK cells were harvested and resuspended in RPMI 1640 supplemented with 10% FCS for cytotoxicity assays using as target cells the Raji cell line. The results are presented in Table 2 where "SD" is the standard deviation. It is believed that high standard deviations reflect donor variability.

9

## TABLE 2

| Example No. or Comparison | PME | Cell Conc. (no./mL) $\times 10^{-6}$ | No. of Donor Samples | Lytic Units mean | SD |
|---|---|---|---|---|---|
| G | no | 1.5 | 10 | 20.1 | 11.8 |
| 10 | no | 1.5 | 10 | 24.4 | 13.8 |
| 11 | yes | 1.5 | 17 | 25.0 | 16.8 |
| 12 | yes | 10 | 19 | 24.4 | 15.1 |

## EXAMPLES 13

These experiments were conducted using procedures similar to those set forth in Examples 10-12. PBMC from six donors were prepared for culture in T-flask (comparisons) or LLDPE bags. None of the PBMC were treated with L-phenylalanine methyl ester. The results are given in Table 3 using Lytic Units as measured by cytotoxicity assays with Raji cells as targets. The abbreviation "nt" indicates that the cells were not tested in the particular device.

## TABLE 3

| Donor No. | Cell Conc. (no./mL) $\times 10^{-6}$ | Lytic Units T-flask | Bags |
|---|---|---|---|
| 1 | 1.0 | 6.3 | nt |
| | 2.0 | 20.0 | nt |
| | 5.0 | 25.0 | nt |
| 2 | 1.0 | 7.7 | 14.3 |
| | 1.5 | 9.5 | 20.8 |
| 3 | 1.0 | 15.2 | 38.5 |
| | 5.0 | 18.5 | 25.0 |
| | 10 | 1.0 | 1.8 |
| | 15 | 0.5 | <0.1 |
| 4 | 3.0 | nt | 34.5 |
| 5 | 5.0 | nt | 25.0 |
| | 10 | nt | 0.7 |
| | 20 | nt | 0.1 |
| 6 | 5.0 | nt | 35.7 |
| | 10 | nt | 1.5 |
| | 20 | nt | 0.3 |

## EXAMPLE 14

Except as otherwise stated these experiments were conducted using procedures similar to those described in Examples 10-12. PBMC from four donors were in each case split into two equal portions. In each case one portion was treated with L-phenylalanine methyl ester (PME) and the other was subjected directly to culturing. Culturing was performed in closed LLDPE bags. The results are presented in Table 4.

### TABLE 4

| Donor No. | Cell Conc. (no./mL) $\times 10^6$ | Lytic Units PME | Untreated |
|-----------|-----------|------|-----------|
| 1 | 12 | 4.7 | 0.7 |
| 2 | 13 | 15.1 | <0.1 |
| 3 | 14 | 15.2 | 2.9 |
| 4 | 15 | 3.6 | 1.7 |

### EXAMPLE 15

Except as otherwise indicated these experiments were conducted using procedures similar to those described in Examples 10-12. PBMC from three donors were used to compare LAK cell activation in T-flask with activation in LLDPE bags at relatively high cell densities, e.g. $1\times10^6$ to $1\times10^7$. The results are presented in Table 5.

### TABLE 5

| Donor No. | Cell Conc. (no./mL) x $10^6$ | PME | Lytic Units T-flask | Bags |
|-----------|-----------|-----|---------|------|
| 1 | 1.0 | no | 2.3 | nt |
|   | 5.0 |    | 4.0 | 27.0 |
|   | 10  |    | 0.1 | nt |
| 2 | 1.0 | yes | 3.7 | nt |
|   | 5.0 |     | 2.9 | 11.9 |
|   | 10  |     | 16.1 | nt |
| 3 | 5 | yes | 3.8 | 18.5 |
|   | 10 |    | 6.9 | nt |

### EXAMPLES 16-18 AND COMPARISON H

Using procedures similar to those described in Examples 1-9 LAK cells are generated by culturing PBMC for four days in bags about 10 in$^2$ in size constructed of a copolymer of (a) ethylene (about 97 mol %) and 1-octene having a density of 0.918 g/cm$^3$, (b) ethylene-methacrylic acid copolymer (Nucrell 0903 polymer, a product of the Du Pont Company), (c) ionomer (Surlyn copolymer, a product of the Du Pont Company), and polyvinyl chloride (PVC) (Comparison). The PBMC cells were present in a concentration of $5\times10^6$ cells/mL and 50 mL of culture fluid were employed. Raji cells were used as target cells. The results are presented in Table 6 where cytotoxicity is indicated in terms of counts per minute.

TABLE 6

| Example No. or Comparison | Bag Thickness, mm (mil) | Material | E:T Ratio | Counts Per Minute |
|---|---|---|---|---|
| 16 | 0.13 (5) | a | 1.25 | 82 |
| | | | 2.5 | 106 |
| | | | 5 | 138 |
| | | | 10 | 206 |
| | | | 20 | 239 |
| | | | 40 | 251 |
| H | 0.36 (14) | PVC | 1.25 | 27 |
| | | | 2.5 | 36 |
| | | | 5 | 57 |
| | | | 10 | 76 |
| | | | 20 | 41 |
| | | | 40 | 32 |
| 17 | 0.13 (5) | b | 1.25 | 52 |
| | | | 2.5 | 55 |
| | | | 5 | 67 |
| | | | 10 | 109 |
| | | | 20 | 145 |
| | | | 40 | 185 |
| 18 | 0.05 (2) | c | 1.25 | 58 |
| | | | 2.5 | 60 |
| | | | 5 | 95 |
| | | | 10 | 133 |
| | | | 20 | 178 |
| | | | 40 | 200 |

**EXAMPLE 19**

Tests in these experiments were run in a film-test chamber permitting the evaluation of films of different compositions and thicknesses. The chamber was constructed on polycarbonate cylinders with an outside diameter of four inches, inside diameter of two inches, and a depth of two inches. The chamber was steam sterilized. Next a radiation-sterilized test film was mechanically clamped over one end and pressure sealed to prevent liquid loss. With the chamber placed film end down, a suspension of cells and media to be cultured were added. A second sterile film of the same composition and thickness as the first was then placed over the top of the chamber and clamped tight to prevent leakage. Using procedures similar to those described in Examples 1-9, PBL were cultured in such chambers.

Tests were run which showed that the chamber was equivalent to a bag run with the same film, cell density and media depth. In a typical run LLDPE film used with the chamber gave an LU30 of 6.5 whereas a bag of the same composition gave 5.4. LAK activation was conducted in these chambers using Surlyn 1601 ionomer films (a product of the Du Pont Company) of 0.05, 0.13, 0.18 and 25 mm (2, 5, 7.5, 10 mil) thicknesses. These films had transmission rates of about 340, 136, 88 and 68 cc/24 hr-atm-100 in$^2$,

respectively. LU30 results were 15, 11, 7.4 and 3 respectively.

**Claims**

1. A process wherein peripheral blood mononuclear cells or lymphocytes from tumor-tissue are cultured to produce a population of cells which are cytotoxic for natural killer cell resistant tumor cells, comprising culturing at a cell concentration of at least about $1.0 \times 10^6$ cells/mL a population of cells selected from the group consisting of peripheral blood mononuclear cells, peripheral blood lymphocytes resulting therefrom, and lymphocytes obtained from tumor tissue in a closed container made from a copolymeric film material having an oxygen permeability of at least about $1.8 \times 10^5$ $\mu m^3$ (STP)/- ($m^2 \cdot sec \cdot Pa$); and a thickness of from about 0.04 mm to about 0.23 mm; said material being selected from the group consisting of

   (a) copolymers of ethylene and an $\alpha$-olefin of 4-10 carbon atoms having a density of from about 0.915-0.925 $g/cm^3$;
   (b) copolymers of ethylene and methacrylic acid;
   (c) ionomers; and
   (d) laminates or coextrudates of an ionomer/polyester elastomer and a linear low density polyethylene elastomer.

2. A process according to Claim 1 wherein the population of cells which is cultured is selected from peripheral blood mononuclear cells and peripheral blood lymphocytes resulting therefrom.

3. A process according to Claim 2 wherein said peripheral blood mononuclear cells or peripheral blood lymphocyte are human cells.

4. A process according to Claim 3 wherein the peripheral blood mononuclear cells or peripheral blood lymphocytes resulting therefrom are cultured for 2-7 days in the presence of recombinant interleukin-2 at a concentration of from about 500 pM to 50,000 pM.

5. A process according to Claim 4 wherein the peripheral blood mononuclear cells or peripheral blood lymphocytes are cultured at a temperature of from about 35° to about 39° C.

6. A process according to Claim 5 wherein the peripheral blood mononuclear cells or peripheral blood lymphocytes are cultured in the presence of from about 3% to about 10% by volume $CO_2$ atmosphere.

7. A process according to Claim 6 wherein peripheral blood lymphocytes are used.

8. A process according to Claim 7 wherein the concentration of peripheral blood lymphocytes is from about $1 \times 10^6$ cells/mL to about $5 \times 10^7$ cells/mL.

9. A process according to Claim 8 wherein the peripheral blood lymphocytes are cultured for 3-5 days.

10. A process according to Claim 9 wherein the recombinant interleukin-2 concentration is from about 1000 pM to about 2000 pM.

11. A process according to Claim 10 wherein the copolymeric film material consists essentially of a copolymer of ethylene and an $\alpha$-olefin of 4-10 carbon atoms, said copolymer having a density of from about 0.915-0.925 $g/cm^3$.

12. A process according to Claim 11 wherein the $\alpha$-olefin of 4-10 carbon atoms is 1-butene or 1-octene and said copolymeric film material has a thickness of from about 0.04 mm to about 0.14 mm.

13. A process according to Claim 12 wherein the $\alpha$-olefin of 4-10 carbon atoms is 1-octene.

14. A process according to Claim 13 wherein the container is closed by heat sealing.

15. A process according to Claim 14 wherein the concentration of peripheral blood lymphocytes is from about $5 \times 10^6$ to about $1.5 \times 10^7$ cells/mL.

**16.** A process according to Claim 15 wherein the peripheral blood lymphocytes are cultured in RPMI 1640 medium supplemented with human serum albumin.

**17.** A process according to any one of claims 1 to 16 wherein, prior to culturing, the peripheral blood mononuclear cells or peripheral blood lymphocytes are contacted with a L-amino acid lower alkyl ester or hydrogen chloride salt thereof, said L-amino acid being selected from the group consisting of phenylalanine, glutamic acid, glutamine and tyrosine and mixtures thereof.

**18.** A process according to Claim 17 wherein the L-amino acid lower alkyl ester is phenylalanine methyl ester.

**Patentansprüche**

**1.** Verfahren, in dem mononukleäre Zellen aus peripherem Blut oder Lymphocyten aus Tumor-Gewebe zur Produktion einer Population von Zellen kultiviert werden, die cytotoxisch für solche Tumor-Zellen sind, die gegenüber natürlichen Killer-Zellen resistent sind, umfassend das Kultivieren einer Population von Zellen, die aus der aus mononukleären Zellen aus peripherem Blut, daraus resultierenden Lymphocyten aus peripherem Blut und aus Tumor-Gewebe erhaltenen Lymphocyten bestehenden Gruppe ausgewählt sind, bei einer Zell-Konzentration von wenigstens etwa $1,0 \times 10^6$ Zellen/ml in einem geschlossenen Behälter, der aus einem copolymeren Filmmaterial mit einer Sauerstoff-Permeabilität von wenigstens etwa $1,8 \times 10^5$ $\mu m^3/m^2 \cdot s \cdot Pa$ (unter Standard-Bedingungen von Druck und Temperatur) und einer Dicke von etwa 0,04 bis etwa 0,023 mm hergestellt ist, wobei das Material aus der aus
    a) Copolymeren aus Ethylen und einem $\alpha$-Olefin mit 4 bis 10 Kohlenstoff-Atomen mit einer Dichte von etwa 0,915 bis 0,925 $g/cm^3$;
    b) Copolymeren aus Ethylen und Methacrylsäure;
    c) Ionomeren; und
    d) Laminaten oder Co-Extrudaten eines Ionomer/Polyester-Elastomers und eines Elastomers aus linearem Polyethylen mit niedriger Dichte
bestehenden Gruppe ausgewählt ist.

**2.** Verfahren nach Anspruch 1, worin die Population von Zellen, die kultiviert wird, aus mononukleären Zellen aus peripherem Blut und daraus resultierenden Lymphocyten aus peripherem Blut ausgewählt ist.

**3.** Verfahren nach Anspruch 2, worin die mononukleären Zellen aus peripherem Blut oder die Lymphocyten aus peripherem Blut menschliche Zellen sind.

**4.** Verfahren nach Anspruch 3, worin die mononukleären Zellen aus peripherem Blut oder die Lymphocyten aus peripherem Blut 2 bis 7 Tage in Gegenwart von rekombiniertem Interleukin-2 in einer Konzentration von etwa 500 pM bis 50 000 pM kultiviert werden.

**5.** Verfahren nach Anspruch 4, worin die mononukleären Zellen aus peripherem Blut oder die Lymphocyten aus peripherem Blut bei einer Temperatur von etwa 35 °C bis etwa 39 °C kultiviert werden.

**6.** Verfahren nach Anspruch 5, worin die mononukleären Zellen aus peripherem Blut oder die Lymphocyten aus peripherem Blut in Gegenwart einer Atmosphäre von etwa 3 Vol.-% bis etwa 10 Vol.-% $CO_2$ kultiviert werden.

**7.** Verfahren nach Anspruch 6, worin Lymphocyten aus peripherem Blut verwendet werden.

**8.** Verfahren nach Anspruch 7, worin die Konzentration der Lymphocyten aus peripherem Blut etwa $1 \times 10^6$ Zellen/ml bis etwa $5 \times 10^7$ Zellen/ml beträgt.

**9.** Verfahren nach Anspruch 8, worin die Lymphocyten aus peripherem Blut 3 bis 5 Tage kultiviert werden.

**10.** Verfahren nach Anspruch 9, worin die Konzentration des rekombinierten Interleukin-2 etwa 1000 pM bis etwa 2000 pM beträgt.

**11.** Verfahren nach Anspruch 10, worin das copolymere Filmmaterial im wesentlichen aus einem Copolymer aus Ethylen und einem α-Olefin mit 4 bis 10 Kohlenstoff-Atomen mit einer Dichte von etwa 0,915 bis 0,925 g/cm³ besteht.

**12.** Verfahren nach Anspruch 11, worin das α-Olefin mit 4 bis 10 Kohlenstoff-Atomen 1-Buten oder 1-Octen ist und das copolymere Filmmaterial eine Dicke von etwa 0,04 mm bis etwa 0,14 mm hat.

**13.** Verfahren nach Anspruch 12, worin das α-Olefin mit 4 bis 10 Kohlenstoff-Atomen 1-Octen ist.

**14.** Verfahren nach Anspruch 13, worin der Behälter durch Heißversiegeln verschlossen wird.

**15.** Verfahren nach Anspruch 14, worin die Konzentration der Lymphocyten aus peripherem Blut etwa 5 x 10⁶ Zellen/ml bis etwa 1,5 x 10⁷ Zellen/ml beträgt.

**16.** Verfahren nach Anspruch 15, worin die Lymphocyten aus peripherem Blut in durch Human-Serumalbumin ergänztem RPMI 1640-Medium kultiviert werden.

**17.** Verfahren nach irgendeinem der Ansprüche 1 bis 16, worin vor dem Kultivieren die mononukleären Zellen aus peripherem Blut oder die Lymphocyten aus peripherem Blut mit L-Aminosäure-Niederalkylester oder einem Hydrochlorid-Salz desselben in Berührung gebracht werden, wobei die Aminosäure aus der aus Phenylalanin, Glutaminsäure, Glutamin und Tyrosin und deren Mischungen bestehenden Gruppe ausgewählt ist.

**18.** Verfahren nach Anspruch 17, worin der L-Aminosäure-Niederalkylester Phenylalaninmethylester ist.

## Revendications

**1.** Un procédé dans lequel des lymphocytes ou des cellules mononucléaires de sang périphérique provenant de tissus tumoraux sont cultivés pour produire une population de cellules qui sont cytotoxiques vis-à-vis des cellules tumorales résistant aux cellules tueuses naturelles, ce procédé comprenant la culture à une concentration de cellules d'au moins environ 1,0x10⁶ cellules/ml d'une population de cellules choisie dans le groupe comprenant des cellules mononucléaires de sang périphérique, des lymphocytes de sang périphérique en résultant, et des lymphocytes obtenus à partir de tissus tumoraux, dans un récipient clos formé d'un film de matière copolymère présentant une perméabilité à l'oxygène d'au moins environ 1,8x10⁵ μm³ (STP)/(m².sec.Pa), et une épaisseur comprise entre environ 0,04 mm et environ 0,23 mm, cette matière étant choisie dans le groupe comprenant:
(a) des copolymères d'éthylène et d'une α-oléfine ayant 4 à 10 atomes de carbone, présentant une densité comprise entre 0,915 et 0,925 g/cm3;
(b) des copolymères d'éthylène et d'acide méthacrylique,
(c) des ionomères; et
(d) des stratifiés ou co-extrudats d'un élastomère à base ionomère/polyester et d'un élastomère formé de polyéthylène linéaire de faible densité.

**2.** Un procédé selon la revendication 1, dans lequel la population de cellules qui est cultivée est choisie parmi des cellules mononucléaires de sang périphérique et des lymphocytes de sang périphérique en résultant.

**3.** Un procédé selon la revendication 2, dans lequel les cellules mononucléaires de sang périphérique et les lymphocytes de sang périphérique sont des cellules humaines.

**4.** Un procédé selon la revendication 3, dans lequel les cellules mononucléaires de sang périphérique et les lymphocytes de sang périphérique en résultant sont cultivés pendant 2 à 7 jours en présence d'interleukine-2 recombinante, à une concentration comprise entre environ 500 pM et 50 000 pM.

**5.** Un procédé selon la revendication 4, dans lequel les cellules mononucléaires de sang périphérique ou les lymphocytes de sang périphérique sont cultivés à une température comprise entre environ 35° et environ 39°C.

**6.** Un procédé selon la revendication 5, dans lequel les cellules mononucléaires de sang périphérique et les lymphocytes de sang périphérique sont cultivés en présence d'une atmosphérique contenant d'environ 3 à environ 10% en volume de $CO_2$.

**7.** Un procédé selon la revendication 6, dans lequel sont employés les lymphocytes de sang périphérique.

**8.** Un procédé selon la revendication 7, dans lequel la concentration en lymphocytes de sang périphérique est comprise entre $1x10^6$ cellules/ml et environ $5x10^7$ cellules/ml.

**9.** Un procédé selon la revendication 8, dans lequel les lymphocytes de sang périphérique sont cultivés durant 3 à 5 jours.

**10.** Un procédé selon la revendication 9, dans lequel la concentration en interleukine-2 recombinante est comprise entre environ 1 000 pM et environ 2 000 pM.

**11.** Un procédé selon la revendication 10, dans lequel le film de matière copolymère est essentiellement forme d'un copolymère d'éthylène et d'une $\alpha$-oléfine ayant 4 à 10 atomes de carbone, ce copolymère présentant une densité comprise entre environ 0,915 et 0,925 g/cm3.

**12.** Un procédé selon la revendication 11, dans lequel l'$\alpha$-oléfine ayant 4 à 10 atomes de carbone consiste en 1-butène ou 1-octène et le film en matière copolymère précité présente une épaisseur comprise entre environ 0,04 mm et environ 0,14 mm

**13.** Un procédé selon la revendication 12, dans lequel l'$\alpha$-olèfine comprenant 4 à 10 atomes de carbone est le 1-octène.

**14.** Un procédé selon la revendication 13, dans lequel le récipient est clos par scellement à chaud.

**15.** Un procédé selon la revendication 14, dans lequel la concentration en lymphocytes de sang périphérique est comprise entre environ $5x10^6$ et environ $1,5x10^7$ cellules/ml.

**16.** Un procédé selon la revendication 15, dans lequel les lymphocytes de sang périphérique sont cultivés dans un milieu formé de RPMI 1640 additionné d'albumine de sérum humain.

**17.** Un procédé selon l'une quelconque des revendications 1 à 16, dans lequel, préalablement à la culture, les cellules mononucléaires de sang périphérique ou les lymphocytes de sang périphérique sont mis au contact d'un ester alkylique inférieur de L-aminoacide ou d'un chlorhydrate en dérivant, cet L-aminoacide étant choisi dans le groupe comprenant phénylalanine, acide glutamique, glutamine, tyrosine et leurs mélanges.

**18.** Un procédé selon la revendication 17, dans lequel l'ester alkylique inférieur de L-aminoacide est l'ester méthylique de phénylalanine.